# EUROPEAN PATENT SPECIFICATION

(11) **EP 3 462 869 B1**
(45) Date of publication and mention of the grant of the patent: **10.01.2024**
(21) Application number: 17728049.2
(22) Date of filing: 17.05.2017
(51) Int. Cl.: A01N 43/08, A01N 43/36, A01N 37/36, A01N 65/22, A01N 59/20, A01P 1/00

(54) **DISINFECTING COMPOSITION COMPRISING TARTARIC ACID AND LACTIC ACID**
DESINFIZIERENDE ZUSAMMENSETZUNG MIT WEINSÄURE UND MILCHSÄURE
COMPOSITION DÉSINFECTANTE COMPRENANT DE L'ACIDE TARTRIQUE ET DE L'ACIDE LACTIQUE

(30) Priority: 02.06.2016 DK 201670395
(43) Date of publication of application: 10.04.2019
(73) Proprietor: Wiping Systems ApS, 2740 Skovlunde (DK)
(72) Inventor: JACOBSSON, Mogens, 2920 Charlottenlund (DK); ESSER, Karlheinz, 41199 Mönchengladbach (DE); LISOWSKY, Thomas, 40789 Monheim (DE)
(74) Representative: Zacco Denmark A/S
(86) International application number: PCT/DK2017/050161
(87) International publication number: WO 2017/207003

(56) References cited:
- EP-A1- 1 146 111
- US-A- 4 900 721
- US-A1- 2009 324 789
- US-A1- 2013 065 959
- US-B2- 6 753 305
- OLMEZ H ET AL: "Potential alternative disinfection methods for organic fresh-cut industry for minimizing water consumption and environmental impact", LWT- FOOD SCIENCE AND TECHNOLOGY, ACADEMIC PRESS, UNITED KINGDOM, vol. 42, no. 3, 1 April 2009 (2009-04-01), pages 686-693, XP025840670, ISSN: 0023-6438, DOI: 10.1016/J.LWT.2008.08.001 [retrieved on 2008-08-14]

## Description

The invention relates to a new highly effective surface disinfecting composition which works under Annex 1 of the biocidal regulation, which means that it is safe for humans and for the environment.

### Background

Contamination with microbes on various surfaces in for example food processing, production facilities, diagnostic laboratories, hygiene institutions, hospitals and in general household cause major problems. Several known disinfection solutions exist on the marked but many of them uses aggressive chemicals to kill the microorganisms. Many such products have the disadvantage of being both corrosive and irritating for the skin of the user and therefore more biocompatible solutions are wanted.

The European Chemical Agency (ECHA) have introduced into the Biocidal Product Regulations (BPR) a section comprising biocides that are considered safe or of little concern. This section of the BPR is called Annex I and comprises a list of 19 chemicals (at the time of filing this application). The listed chemicals in Annex I are outside the conventional biocides listed in Annex II of the BPR.

Annex I of the Biocidal Products Regulation (BPR) lists initially active substances identified as presenting a low risk under Regulation (EC) No 1907/2006 or Directive 98/8/EC, substances identified as food additives, pheromones and other substances considered to have low toxicity, such as weak acids, alcohols and vegetable oils used in cosmetics and food. To encourage the use of products with a more favourable environmental, human or animal health profile, biocidal products containing one or more of these active substance(s) are eligible for a simplified authorisation procedure.

WO 2012/101129 A1 discloses an anti-microbial composition comprising essential oils, metal ions, organic acids and detergents. Several of the ingredients of choice in WO 2012/101129 A1 are not listed in Annex I of the BPR and are therefore not as green and safe as the chemicals listed in Annex I. Other anti-microbial compositions are known from e.g. US6753305 B2, US4900721 A, US2009/324789 A1, EP1146111 A1 and US2013/065959 A1.

However, there is a need to develop disinfecting products that are safe for both humans and for the environment, but without compromising to the demand of performance.

### Summary of the invention

The object of the present invention is to solve the above described problems by developing a new disinfecting composition that are completely safe for both humans and for the environment and which have a very high antimicrobial efficiency.

This is accomplished by a disinfecting composition comprising the following biocides:
a) Tartaric acid in concentrations from 1 mM to 70 mM;
b) Lactic acid in concentrations from 1 mM to 70 mM,
c)
   1) lavender oil or lavender oil extract and
   2) peppermint oil or peppermint oil extract in concentrations from 0.01 wt% to 1 wt%,
d) copper-PCA in concentrations from 1 mM to 10 mM.

The claimed composition has shown to be a broad-spectrum powerful disinfectant comprising the weak natural organic acids - tartaric acid and lactic acid - that are even approved for indirect contact with food. The composition works under Annex 1 of the Biocidal Products Regulation (BPR), which means it is safe for humans and environment so the composition won't have a hazard warning and it is effective in killing bacteria and candida which is a yeast microorganism that lives in the body's intestinal tract, and also effective in killing bacteria, fungi, viruses, mycobacteria and Clostridium difficile. In fact the claimed disinfecting composition will cover more than 93% of all situations replacing several conventional disinfectants such as ethanol and carcinogenic PHMB.

Also disclosed but not covered by the scope of protection is a disinfecting composition comprising the following biocides
a) Tartaric acid in concentrations from 1 mM to 70 mM;
b) Lactic acid in concentrations from 1 mM to 70 mM,
c) at least one essential oil or oil extract in concentrations from 0.01 wt% to 1 wt% selected from lavender oil or oil extract and/or peppermint oil or oil extract,
d) copper-PCA (Cu-PCA) in concentrations from 1 mM to 10 mM,

Cu-PCA is an enhancer used in the cosmetic industry that conditions and clears the skin. Cu-PCA is an anti-irritant and skin conditioner which has positive effects on collagen and elastin in the skin, which helps cell regeneration and skin repair in wound healing.

Also disclosed but not covered by the scope of protection is a disinfecting composition comprising the following biocides
a) Tartaric acid in concentrations from 1 mM to 70 mM;
b) Lactic acid in concentrations from 1 mM to 70 mM,
c) at least one essential oil or oil extract in concentrations from 0.01 wt% to 1 wt% selected from lavender oil or oil extract and/or peppermint oil or oil extract,
d) copper-PCA in concentrations from 1 mM to 10 mM,
e) Vitamin C (L-ascorbic acid) in concentrations from 1 mM to 50 mM.

For outbreaks the composition comprising L-ascorbic acid or vitamin C can replace chlorine, peroxide compounds, glutaraldehyde etc. When the disinfecting composition is boosted with Vitamin C it increases the kill power of the disinfecting composition and the composition is able to kill 100% of pathogens. It can be used in critical areas, i.e. by clinical outbreaks, lumened endoscope disinfection and virulent black mould.

This disinfecting composition comprising L-ascorbic acid or vitamin C has a very high antimicrobial efficiency and also works under Annex 1 of the Biocidal Products Regulation (BPR), which means it is safe for humans and the environment so the composition won't have a hazard warning. It is effective in killing bacteria, fungi, viruses, mycobacteria and spores incl. Aspergillus Brasiliensis, Clostridium difficile, Bacillus Subtilis and Bacillus Cereus.

The present invention also concerns the use of the disinfecting composition according to the present claims for treating contaminated surfaces where the surfaces is selected from surfaces on fixed panel equipment and non-rinse/washable equipment such as control panels, medical instruments such as infusion pumps, stethoscopes, thermometers, blood pressure-apparatus, nanometres or flow meters, beds, nursing pillow, food preparation surfaces and equipment, walls or floors. Such surfaces may be at children institutions, at hospitals, at nursing homes, at clinics, in food and beverage processing, in agricultural industry, in farming, in animal biosecurity, in consumer market, in food catering, in brewing industry, in oil industry, in pharmaceutical industry, in cleaning and disinfectant industry, in electronic industry and in fishing industry.

Further disclosed but not covered by the scope of protection is a method for disinfecting a contaminated surface, said method may comprising:
- providing a disinfecting composition comprising tartaric acid in concentrations from 1 mM to 70 mM and lactic acid in concentrations from 1 mM to 70 mM;
- treating a contaminated surface with said disinfecting composition, wherein bacteria and yeast microorganism candida on said surface is killed by denaturing, solubilising inactivating and degrading their proteins and nucleic acids.

Further disclosed but not covered by the scope of protection is a method for disinfecting a contaminated surface, said method may comprising:
- providing a disinfecting composition comprising tartaric acid in concentrations from 1 mM to 70 mM; lactic acid in concentrations from 1 mM to 70 mM and at least one essential oil or oil extract in concentrations from 0.01 wt% to 1 wt% selected from lavender oil or oil extract and/or peppermint oil or oil extract;
- treating a contaminated surface with said disinfecting composition, wherein microorganisms on said surface is killed by denaturing, solubilising inactivating and degrading their proteins and nucleic acids.

Further disclosed but not covered by the scope of protection is a method for disinfecting a contaminated surface, said method may comprising:
- providing a disinfecting composition comprising tartaric acid in concentrations from 1 mM to 70 mM, lactic acid in concentrations from 1 mM to 70 mM, at least one essential oil or oil extract in concentrations from 0.01 wt% to 1 wt% selected from lavender oil or oil extract and/or peppermint oil or oil extract and copper-PCA in concentrations from 1 mM to 10 mM;
- treating a contaminated surface with said disinfecting composition, wherein microorganisms on said surface is killed by denaturing, solubilising inactivating and degrading their proteins and nucleic acids.

Further disclosed but not covered by the scope of protection is a method for disinfecting a contaminated surface, said method may comprising:
- providing a disinfecting composition comprising tartaric acid in concentrations from 1 mM to 70 mM, lactic acid in concentrations from 1 mM to 70 mM, at least one essential oil or oil extract in concentrations from 0.01 wt% to 1 wt% selected from lavender oil or oil extract and/or peppermint oil or oil extract, copper-PCA in concentrations from 1 mM to 10 mM and L-ascorbic acid in concentrations from 1 mM to 50 mM;
- treating a contaminated surface with said disinfecting composition, wherein microorganisms on said surface is killed by denaturing, solubilising inactivating and degrading their proteins and nucleic acids.

### Brief description of the drawings

Figure 1 show that the composition of the present invention is able to target the following organisms: gram positive and gram negative bacteria, yeast and fungi, enveloped and non-enveloped viruses, mycobacteria and bacterial spores.
Figure 2 shows the contact time of the composition of the present invention against varies type of bacteria, fungi viruses and spores.

### Description of preferred embodiments

The present invention in a first aspect provides a disinfecting composition comprising the following biocides:
a) Tartaric acid in concentrations from 1 mM to 70 mM;
b) Lactic acid in concentrations from 1 mM to 70 mM,
c)
   1) lavender oil or lavender oil extract and
   2) peppermint oil or peppermint oil extract in concentrations from 0.01 wt% to 1 wt%,
d) copper-PCA in concentrations from 1 mM to 10 mM.

Also disclosed but not covered by the scope of protection is a disinfecting composition comprising the following biocides
a) Tartaric acid in concentrations from 1 mM to 70 mM;
b) Lactic acid in concentrations from 1 mM to 70 mM.

It was surprisingly found that this composition, comprising the mixture of tartaric acid and lactic acid is effective in killing bacteria and candida - which is a yeast microorganism that lives in the body's intestinal tract and this new disinfecting composition is completely safe for both humans and for the environment

In one aspect not covered by the scope of protection the disinfecting composition further comprises the following biocide:
c) at least one essential oil or oil extract in concentrations from 0.01 wt% to 1 wt% selected from lavender oil or oil extract and/or peppermint oil or oil extract.

It was surprisingly found that this composition, comprising the mixture of tartaric acid, lactic acid and at least one essential oil or oil extract selected from lavender oil and/or peppermint oil exhibit a high antimicrobial activity. This new disinfecting composition is completely safe for both humans and for the environment and has a very high antimicrobial efficiency.

The claimed composition has shown to be a broad-spectrum powerful disinfectant comprising the weak natural organic acids - tartaric acid and lactic acid - that are even approved for indirect contact with food. The composition works under Annex 1 of the Biocidal Products Regulation (BPR), which means it is safe for humans and environment so the composition won't have a hazard warning and it is effective in killing bacteria, fungi, viruses, mycobacteria and Clostridium difficile. In fact the claimed disinfecting composition will cover more than 93% of all situations replacing several conventional disinfectants such as ethanol and carcinogenic PHMB.

In one aspect not covered by the scope of protection the disinfecting composition comprising the following biocides:
a) Tartaric acid in concentrations from 1 mM to 70 mM;
b) Lactic acid in concentrations from 1 mM to 70 mM,
c) at least one essential oil or oil extract in concentrations from 0.01 wt% to 1 wt% selected from lavender oil or oil extract and/or peppermint oil or oil extract,
d) copper-PCA (Cu-PCA) in concentrations from 1 mM to 10 mM.

Cu-PCA is an enhancer used in the cosmetic industry that conditions and clears the skin.

In one aspect not covered by the scope of protection the disinfecting composition comprising the following biocides:
a) Tartaric acid in concentrations from 1 mM to 70 mM;
b) Lactic acid in concentrations from 1 mM to 70 mM,
c) at least one essential oil or oil extract in concentrations from 0.01 wt% to 1 wt% selected from lavender oil or oil extract and/or peppermint oil or oil extract,
d) copper-PCA (Cu-PCA) in concentrations from 1 mM to 10 mM,
e) Vitamin C (L-ascorbic acid) in concentrations from 1 mM to 50 mM.

For outbreaks the composition comprising L-ascorbic acid or vitamin C can replace chlorine, peroxide compounds, glutaraldehyde etc. When the disinfecting composition is boosted with Vitamin C it increases the kill power of the disinfecting composition and the composition is able to kill 100% of pathogens. It can be used in critical areas, i.e. by clinical outbreaks, lumened endoscope disinfection and virulent black mould.

The disinfecting composition comprising L-ascorbic acid or vitamin C has a very high antimicrobial efficiency and also works under Annex 1 of the Biocidal Products Regulation (BPR), which means it is safe for humans and the environment so the composition won't have a hazard warning. It is effective in killing bacteria, fungi, viruses, mycobacteria and spores incl. Aspergillus Brasiliensis, Clostridium difficile, Bacillus Subtilis and Bacillus Cereus.

The claimed disinfecting composition can be formulated into wipes or as a fluid. The microorganisms are killed with high efficiency by rubbing, wiping, spraying, soaking or immersion or in any other ways applying the composition solution to a contaminated surface contaminated with unwanted microorganisms.

Advantages of the disinfecting composition according to the present invention are:
- High efficacy
- Can be used without gloves
- No hazard warnings - handle as regular waste: Environment friendly
- Safe transportation and storage (and not highly flammable as opposed to Ethanol)
- Approved for surfaces with indirect food-contact, which means no cleaning after disinfection
- Non-corrosive
- No discoloration
- Use on all impervious hard surfaces and equipment with a high frequency (and is safe enough to use several times a day)
- Can be used in all environments - i.e. hospitals, health institutions, food industry and animal farms
- No harmful residuals left on surfaces

The composition can be used on all kind of surfaces, including sensitive equipment i.e. fixed panel equipment and other kinds of non-rinse/washable equipment (i.e. control panels), medical instruments such as infusion pumps, stethoscopes, thermometers, blood pressure-apparatus, nanometres, flow meters, beds, nursing pillow etc., non-alcohol tolerant furniture surfaces, food preparation surfaces and equipment, walls, floors and many other hard surfaces.

The composition can also be used on skin, hands, body, plants, fruits and other foods.

In one aspect not covered by the scope of protection of the disinfecting composition the concentration of the at least one essential oil or oil extract is from 0.05 wt% to 2 wt%, or from 0.1 wt% to 1 wt%, or from 0.1 wt% to 0.5 wt%.

In one aspect not covered by the scope of protection the composition comprises from 0.01 wt% to 0.5 wt%, or from 0.05 wt% to 0.2 wt%, or from 0.09 wt% to 0.1 wt% lavender oil or oil extract and from 0.01 wt% to 0.5 wt%, or from 0.05 wt% to 0.2 wt%, or from 0.09 wt% to 0.1 wt% peppermint oil or oil extract.

Some essential oils are known to exhibit an antiseptic activity; especially tea tree oil is known to work effectively against bacteria and fungi. But many essential oils are not approved and incorporated into the Annex I of the Biocidal Products Regulation (BPR). It is surprisingly found that both lavender oil and extract and peppermint oil and extract have very good antimicrobial activity, and when combined the antimicrobial activity is enhanced.

Both tartaric acid and lactic acid are carboxylic acids and they may be used as either solid or liquid in their natural state and are readily soluble or dissolved in or miscible with water or an aqueous solvent. In one embodiment tartaric acid is used as solid and lactic acid as liquid or solid.

In one embodiment of the disinfecting composition the concentration of the tartaric acid is from 10 mM to 50 mM, or from 20 mM to 50 mM, or from 30 mM to 50 mM, or from 35 mM to 45 mM, or around 40 mM.

In one embodiment of the disinfecting composition the concentration of the tartaric acid is from 1 mM to 30 mM, or from 5 mM to 20 mM, or from 5 mM to 15 mM, or from 5 mM to 10 mM.

In one embodiment of the disinfecting composition the concentration of the lactic acid is from 10 mM to 50 mM, or from 20 mM to 50 mM, or from 30 mM to 50 mM, or from 35 mM to 45 mM, or around 40 mM.

In one embodiment of the disinfecting composition the concentration of the lactic acid is from 1 mM to 30 mM, or from 5 mM to 20 mM, or from 5 mM to 15 mM, or from 5 mM to 10 mM.

In one embodiment the disinfecting composition further comprises at least one buffer substances. Usable buffer substance can be sodium acetate or sodium lactate. The buffer substances can be added to the composition solution to define a specific pH value.

In one embodiment the disinfecting composition further comprises alcohols. In one embodiment the alcohol is Ethanol. The concentration of the alcohol is low compared to other disinfecting compositions. In one embodiment the concentration of alcohol is below 10% wt%, such as below 8% wt% in relation to the total volume of the composition solution. The alcohol will ease a better solubility of the essential oil(s) and a better wetting of the surfaces to be treated.

In one embodiment the disinfecting composition further comprises an anionic surfactant which is stable at pH 2.5 to 4. In one embodiment the anionic surfactant is sodium dodecyl sulfate (SDS). SDS is a widely used detergent which is stable in the pH range of 2.5 to 4. The surface-active substance are preferably used in concentrations from 0.01 wt% to 10 wt%, or from 0.01 wt% to 2 wt%, or from 0.1 wt% to 1 wt%, or from 0.2 wt% to 0.8 wt%, or about 0.5 wt% in relation to the total volume of the composition solution.

In one embodiment the disinfecting composition further comprises glycerol in small amount, such as below 4% wt% or below 1% wt% in relation to the total volume of the composition solution. The glycerol will work as a softening agent. In one embodiment the disinfecting composition further comprises PnB (1-butoxypropan-2-ol) which is a water soluble solvent. If present, the PnB is preferably used in concentrations from 0.01 wt% to 10 wt%, or from 0.1 wt% to 6 wt%, or from 1 wt% to 5 wt% in relation to the total volume of the composition solution.

In one aspect not covered by the scope of protection the disinfecting composition further comprises copper ions e.g. from copper salts.

The disinfecting composition according to claim 1 comprises Cu-PCA, which is an enhancer used in the cosmetic industry that conditions and clears the skin. Cu-PCA is used in concentrations from 1 mM to 10 mM, or from 2 mM to 5 mM, or from 2.5 mM to 3.5 mM, or around 3 mM in relation to the total volume of the composition solution. In one embodiment the disinfecting composition could comprise copper compounds besides Cu-PCA such as Cu-chlorid and/or Cu-sulphate.

In one embodiment, the combination of Cu-PCA and glycerol was found to be effective in regards to skin biocompatibility, without detracting from the overall efficacy performance, even in low concentrations.

In one embodiment the disinfecting composition is having a pH value in the range of 2 to 4, or from 2.5 to 4, or from 2.5 to 3. Within this range the disinfecting effect is optimal, so it is an advantage of the disinfecting composition to have an acidic pH value. In basic solutions, the disinfecting effect of the essential oils is reduced. The pH value can be adjusted within the above ranges by varying the concentration of the organic acids or by adding a chemical suitable for pH adjustment.

In one aspect not covered by the scope of protection the disinfecting composition comprises the following biocides:
a) from 10 mM to 50 mM tartaric acid;
b) from 10 mM to 50 mM lactic acid;
c) from 0.05 wt% to 0.2 wt% lavender oil or oil extract and from 0.05 wt% to 0.2 wt% peppermint oil or oil extract.

In one aspect not covered by the scope of protection the disinfecting composition comprises the following biocides:
a) from 30 mM to 50 mM tartaric acid;
b) from 30 mM to 50 mM lactic acid;
c) from 0.05 wt% to 0.2 wt% lavender oil or oil extract and from 0.05 wt% to 0.2 wt% peppermint oil or oil extract.

In one aspect not covered by the scope of protection the disinfecting composition comprises the following biocides:
a) from 30 mM to 50 mM tartaric acid;
b) from 30 mM to 50 mM lactic acid;
c) from 0.09 wt% to 0.1 wt% lavender oil or oil extract and from 0.09 wt% to 0.1 wt% peppermint oil or oil extract.

In one aspect not covered by the scope of protection the disinfecting composition comprises the following biocides:
a) about 40 mM tartaric acid;
b) about 40 mM lactic acid;
c) from 0.09 wt% to 0.1 wt% lavender oil or oil extract and from 0.09 wt% to 0.1 wt% peppermint oil or oil extract.

In one embodiment the disinfecting composition further comprises at least one water-soluble vitamin. The at least one water-soluble vitamin is included in concentrations from 1 mM to 50 mM. In one embodiment the at least one water-soluble vitamin is vitamin C (L-ascorbic acid). In one embodiment the disinfecting composition comprises vitamin C in concentrations from 1 mM to 50 mM.

So in one embodiment the disinfecting composition further comprises L-ascorbic acid. That is; the disinfecting composition can comprise L-ascorbic acid itself or vitamin C which comprises L-ascorbic acid. Hence, throughout the description; vitamin C and L-ascorbic acid are used interchangeably.

In one embodiment the disinfecting composition comprises L-ascorbic acid in concentrations from 1 mM to 50 mM.

In one embodiment the disinfecting composition further comprises the water-soluble vitamin C (L-ascorbic acid). In one embodiment the vitamin C is included in concentrations from 10 mM to 50 mM, or from 20 mM to 50 mM, or from 30 mM to 50 mM, or from 35 mM to 45 mM, or around 40 mM. In another embodiment the vitamin C is included in concentrations from 1 mM to 30 mM, or from 5 mM to 30 mM, or from 10 mM to 25 mM, or around 20 mM.

In one embodiment the disinfecting composition further comprises L-ascorbic acid. In one embodiment the L-ascorbic acid is included in concentrations from 10 mM to 50 mM, or from 20 mM to 50 mM, or from 30 mM to 50 mM, or from 35 mM to 45 mM, or around 40 mM. In another embodiment the L-ascorbic acid is included in concentrations from 1 mM to 30 mM, or from 5 mM to 30 mM, or from 10 mM to 25 mM, or around 20 mM.

In addition to the at least one water-soluble vitamin; vitamin C or L-ascorbic acid the composition may also comprise NaCl. In one embodiment the NaCl is included in concentrations from 1 mM to 30 mM, or from 5 mM to 20 mM, or from 8 mM to 12 mM, or around 10 mM. In another embodiment the NaCl is included in concentrations from 1 mM to 20 mM, or from 2 mM to 15 mM, or from 3 mM to 10 mM, or around 5 mM.

For outbreaks the composition comprising L-ascorbic acid/vitamin C can replace chlorine, peroxide compounds, glutaraldehyde etc. When the disinfecting composition is boosted with L-ascorbic acid/Vitamin C it increases the kill power of the disinfecting composition and the composition is able to kill 100% of pathogens. It can be used in critical areas, i.e. by clinical outbreaks, lumened endoscope disinfection and virulent black mould.

This disinfecting composition comprising L-ascorbic acid/vitamin C has a very high antimicrobial efficiency and also works under Annex 1 of the Biocidal Products Regulation (BPR), which means it is safe for humans and the environment so the composition won't have a hazard warning. It is effective in killing bacteria, fungi, viruses, mycobacteria and spores incl. Aspergillus Brasiliensis, Clostridium difficile, Bacillus Subtilis and Bacillus Cereus.

The claimed disinfecting composition comprising L-ascorbic acid/vitamin C can be formulated into wipes or as a fluid. The microorganisms are killed with high efficiency by rubbing, wiping, spraying, soaking or immersion or in any other ways applying the composition solution to a contaminated surface contaminated with unwanted microorganisms.

In one aspect not covered by the scope of protection the disinfecting composition comprises the following biocides:
a) from 10 mM to 50 mM tartaric acid;
b) from 10 mM to 50 mM lactic acid;
c) from 0.05 wt% to 0.2 wt% lavender oil or oil extract and from 0.05 wt% to 0.2 wt% peppermint oil or oil extract;
d) from 10 mM to 50 mM L-ascorbic acid or vitamin C.

In one aspect not covered by the scope of protection the disinfecting composition comprises the following biocides:
a) from 30 mM to 50 mM tartaric acid;
b) from 30 mM to 50 mM lactic acid;
c) from 0.05 wt% to 0.2 wt% lavender oil or oil extract and from 0.05 wt% to 0.2 wt% peppermint oil or oil extract;
d) from 30 mM to 50 mM L-ascorbic acid or Vitamin C.

In one aspect not covered by the scope of protection the disinfecting composition comprises the following biocides:
a) from 30 mM to 50 mM tartaric acid;
b) from 30 mM to 50 mM lactic acid;
c) from 0.09 wt% to 0.1 wt% lavender oil or oil extract and from 0.09 wt% to 0.1 wt% peppermint oil or oil extract;
d) from 35 mM to 45 mM L-ascorbic acid or vitamin C.

In one aspect not covered by the scope of protection the disinfecting composition comprises the following biocides:
a) about 40 mM tartaric acid;
b) about 40 mM lactic acid;
c) from 0.09 wt% to 0.1 wt% lavender oil or oil extract and from 0.09 wt% to 0.1 wt% peppermint oil or oil extract;
d) about 40 mM L-ascorbic acid or vitamin C.

In one aspect not covered by the scope of protection the disinfecting composition comprises the following biocides:
a) from 10 mM to 50 mM tartaric acid;
b) from 10 mM to 50 mM lactic acid;
c) from 0.05 wt% to 0.2 wt% lavender oil or oil extract and from 0.05 wt% to 0.2 wt% peppermint oil or oil extract;
d) from 1 mM to 30 mM L-ascorbic acid or vitamin C.

In one aspect not covered by the scope of protection
the disinfecting composition comprises the following biocides:
a) from 30 mM to 50 mM tartaric acid;
b) from 30 mM to 50 mM lactic acid;
c) from 0.05 wt% to 0.2 wt% lavender oil or oil extract and from 0.05 wt% to 0.2 wt% peppermint oil or oil extract;
d) from 5 mM to 30 mM L-ascorbic acid or Vitamin C.

In one aspect not covered by the scope of protection the disinfecting composition comprises the following biocides:
a) from 30 mM to 50 mM tartaric acid;
b) from 30 mM to 50 mM lactic acid;
c) from 0.09 wt% to 0.1 wt% lavender oil or oil extract and from 0.09 wt% to 0.1 wt% peppermint oil or oil extract;
d) from 10 mM to 25 mM L-ascorbic acid or vitamin C.

In one aspect not covered by the scope of protection the disinfecting composition comprises the following biocides:
a) about 40 mM tartaric acid;
b) about 40 mM lactic acid;
c) from 0.09 wt% to 0.1 wt% lavender oil or oil extract and from 0.09 wt% to 0.1 wt% peppermint oil or oil extract;
d) about 20 mM L-ascorbic acid or vitamin C.

The claimed disinfecting composition according to claim 1 comprises Cu-PCA, which is an enhancer used in the cosmetic industry that conditions and clears the skin. Cu-PCA is used in concentrations from 1 mM to 10 mM, or from 2 mM to 5 mM, or from 2.5 mM to 3.5 mM, or around 3 mM in relation to the total volume of the composition solution. In one embodiment the disinfecting composition could comprise copper compounds besides Cu-PCA such as Cu-chlorid and/or Cu-sulphate.

In one embodiment the disinfecting composition comprises the following biocides:
a) from 10 mM to 50 mM tartaric acid;
b) from 10 mM to 50 mM lactic acid;
c) from 0.05 wt% to 0.2 wt% lavender oil or oil extract and from 0.05 wt% to 0.2 wt% peppermint oil or oil extract;
d) from 1 mM to 10 mM copper-PCA.

In one embodiment the disinfecting composition comprises the following biocides:
a) from 30 mM to 50 mM tartaric acid;
b) from 30 mM to 50 mM lactic acid;
c) from 0.05 wt% to 0.2 wt% lavender oil or oil extract and from 0.05 wt% to 0.2 wt% peppermint oil or oil extract;
d) from 1 mM to 10 mM copper-PCA.

In one embodiment the disinfecting composition comprises the following biocides:
a) from 30 mM to 50 mM tartaric acid;
b) from 30 mM to 50 mM lactic acid;
c) from 0.09 wt% to 0.1 wt% lavender oil or oil extract and from 0.09 wt% to 0.1 wt% peppermint oil or oil extract;
d) from 1 mM to 10 mM copper-PCA.

In one embodiment the disinfecting composition comprises the following biocides:
a) about 40 mM tartaric acid;
b) about 40 mM lactic acid;
c) from 0.09 wt% to 0.1 wt% lavender oil or oil extract and from 0.09 wt% to 0.1 wt% peppermint oil or oil extract;
d) from 1 mM to 10 mM copper-PCA.

In one embodiment the disinfecting composition comprises the following biocides:
a) from 10 mM to 50 mM tartaric acid;
b) from 10 mM to 50 mM lactic acid;
c) from 0.05 wt% to 0.2 wt% lavender oil or oil extract and from 0.05 wt% to 0.2 wt% peppermint oil or oil extract;
d) from 1 mM to 10 mM copper-PCA.

In one embodiment the disinfecting composition comprises the following biocides:
a) from 30 mM to 50 mM tartaric acid;
b) from 30 mM to 50 mM lactic acid;
c) from 0.05 wt% to 0.2 wt% lavender oil or oil extract and from 0.05 wt% to 0.2 wt% peppermint oil or oil extract;
d) from 1 mM to 10 mM copper-PCA.

In one embodiment the disinfecting composition comprises the following biocides:
a) from 30 mM to 50 mM tartaric acid;
b) from 30 mM to 50 mM lactic acid;
c) from 0.09 wt% to 0.1 wt% lavender oil or oil extract and from 0.09 wt% to 0.1 wt% peppermint oil or oil extract;
d) from 2 mM to 5 mM copper-PCA.

In one embodiment the disinfecting composition comprises the following biocides:
a) about 40 mM tartaric acid;
b) about 40 mM lactic acid;
c) from 0.09 wt% to 0.1 wt% lavender oil or oil extract and from 0.09 wt% to 0.1 wt% peppermint oil or oil extract;
d) from 2 mM to 5 mM copper-PCA.

In one embodiment the disinfecting composition comprises the following biocides:
a) from 10 mM to 50 mM tartaric acid;
b) from 10 mM to 50 mM lactic acid;
c) from 0.05 wt% to 0.2 wt% lavender oil or oil extract and from 0.05 wt% to 0.2 wt% peppermint oil or oil extract;
d) from 10 mM to 50 mM L-ascorbic acid or vitamin C
e) from 1 mM to 10 mM copper-PCA.

In one embodiment the disinfecting composition comprises the following biocides:
a) from 30 mM to 50 mM tartaric acid;
b) from 30 mM to 50 mM lactic acid;
c) from 0.05 wt% to 0.2 wt% lavender oil or oil extract and from 0.05 wt% to 0.2 wt% peppermint oil or oil extract;
d) from 30 mM to 50 mM L-ascorbic acid or Vitamin C
e) from 1 mM to 10 mM copper-PCA.

In one embodiment the disinfecting composition comprises the following biocides:
a) from 30 mM to 50 mM tartaric acid;
b) from 30 mM to 50 mM lactic acid;
c) from 0.09 wt% to 0.1 wt% lavender oil or oil extract and from 0.09 wt% to 0.1 wt% peppermint oil or oil extract;
d) from 35 mM to 45 mM L-ascorbic acid or vitamin C
e) from 1 mM to 10 mM copper-PCA.

In one embodiment the disinfecting composition comprises the following biocides:
a) about 40 mM tartaric acid;
b) about 40 mM lactic acid;
c) from 0.09 wt% to 0.1 wt% lavender oil or oil extract and from 0.09 wt% to 0.1 wt% peppermint oil or oil extract;
d) about 40 mM L-ascorbic acid or vitamin C
e) from 1 mM to 10 mM copper-PCA.

In one embodiment the disinfecting composition comprises the following biocides:
a) from 10 mM to 50 mM tartaric acid;
b) from 10 mM to 50 mM lactic acid;
c) from 0.05 wt% to 0.2 wt% lavender oil or oil extract and from 0.05 wt% to 0.2 wt% peppermint oil or oil extract;
d) from 1 mM to 30 mM L-ascorbic acid or vitamin C
e) from 1 mM to 10 mM copper-PCA.

In one embodiment the disinfecting composition comprises the following biocides:
a) from 30 mM to 50 mM tartaric acid;
b) from 30 mM to 50 mM lactic acid;
c) from 0.05 wt% to 0.2 wt% lavender oil or oil extract and from 0.05 wt% to 0.2 wt% peppermint oil or oil extract;
d) from 5 mM to 30 mM L-ascorbic acid or Vitamin C
e) from 1 mM to 10 mM copper-PCA.

In one embodiment the disinfecting composition comprises the following biocides:
a) from 30 mM to 50 mM tartaric acid;
b) from 30 mM to 50 mM lactic acid;
c) from 0.09 wt% to 0.1 wt% lavender oil or oil extract and from 0.09 wt% to 0.1 wt% peppermint oil or oil extract;
d) from 10 mM to 25 mM L-ascorbic acid or vitamin C
e) from 2 mM to 5 mM copper-PCA.

In one embodiment the disinfecting composition comprises the following biocides:
a) about 40 mM tartaric acid;
b) about 40 mM lactic acid;
c) from 0.09 wt% to 0.1 wt% lavender oil or oil extract and from 0.09 wt% to 0.1 wt% peppermint oil or oil extract;
d) about 20 mM L-ascorbic acid or vitamin C.
e) from 2 mM to 5 mM copper-PCA.

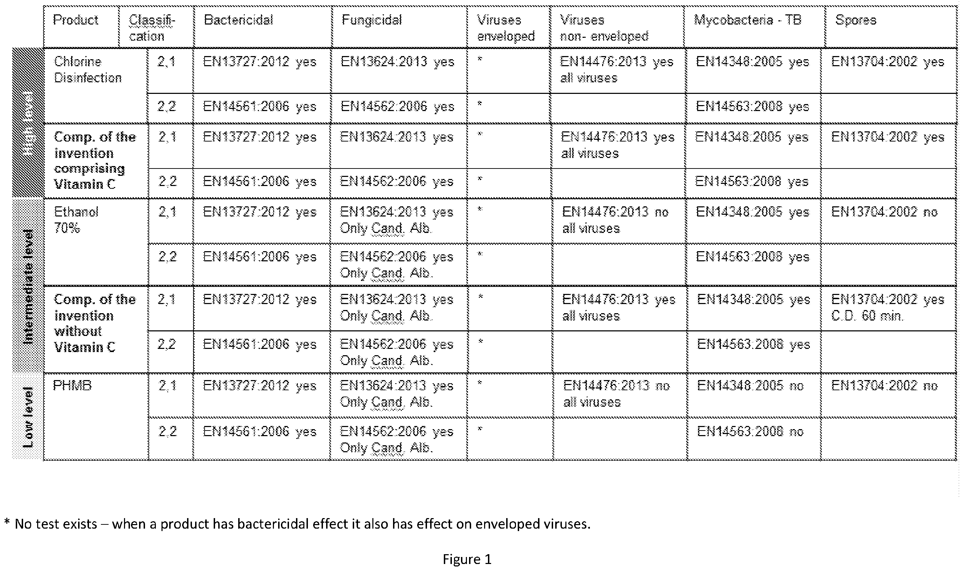

The presently claimed disinfecting composition is aimed at targeting the following microorganisms: gram positive and gram negative bacteria, yeast and fungi, enveloped and non-enveloped viruses, mycobacteria and bacterial spores, please see figure 1.

The disinfecting composition can be used for treating contaminated surfaces such as at children institutions, at hospitals, at nursing homes, at clinics, in food and beverage processing, in agricultural industry, in farming, in animal biosecurity, in consumer market, in food catering, in brewing industry, in oil industry, in pharmaceutical industry, in cleaning and disinfectant industry, in electronic industry and in fishing industry.

The composition can be used on all kind of surfaces, including sensitive equipment i.e. fixed panel equipment and other kinds of non-rinse/washable equipment (i.e. control panels), medical instruments such as infusion pumps, stethoscopes, thermometers, blood pressure-apparatus, nanometres, flow meters, beds, nursing pillow etc., non-alcohol tolerant furniture surfaces, food preparation surfaces and equipment, walls, floors and many other hard surfaces.

The composition can also be used on skin, hands, body, plants, fruits and other foods.

Also disclosed but not covered by the scope of protection is a method for disinfecting a contaminated surface, said method may comprising:
- providing a disinfecting composition comprising tartaric acid in concentrations from 1 mM to 70 mM and lactic acid in concentrations from 1 mM to 70 mM;
- treating a contaminated surface with said disinfecting composition, wherein bacteria and yeast microorganism candida on said surface is killed by denaturing, solubilising inactivating and degrading their proteins and nucleic acids.

Also disclosed but not covered by the scope of protection is a method for disinfecting a contaminated surface, said method may comprising:
- providing a disinfecting composition comprising tartaric acid in concentrations from 1 mM to 70 mM; lactic acid in concentrations from 1 mM to 70 mM and at least one essential oil or oil extract in concentrations from 0.01 wt% to 1 wt% selected from lavender oil or oil extract and/or peppermint oil or oil extract;
- treating a contaminated surface with said disinfecting composition, wherein microorganisms on said surface is killed by denaturing, solubilising inactivating and degrading their proteins and nucleic acids.

Also disclosed but not covered by the scope of protection is a method for disinfecting a contaminated surface, said method may comprising:
- providing a disinfecting composition comprising tartaric acid in concentrations from 1 mM to 70 mM, lactic acid in concentrations from 1 mM to 70 mM, at least one essential oil or oil extract in concentrations from 0.01 wt% to 1 wt% selected from lavender oil or oil extract and/or peppermint oil or oil extract and copper-PCA in concentrations from 1 mM to 10 mM;
treating a contaminated surface with said disinfecting composition, wherein microorganisms on said surface is killed by denaturing, solubilising inactivating and degrading their proteins and nucleic acids.

Also disclosed but not covered by the scope of protection is a method for disinfecting a contaminated surface, said method may comprising:
- providing a disinfecting composition comprising tartaric acid in concentrations from 1 mM to 70 mM, lactic acid in concentrations from 1 mM to 70 mM, at least one essential oil or oil extract in concentrations from 0.01 wt% to 1 wt% selected from lavender oil or oil extract and/or peppermint oil or oil extract, copper-PCA in concentrations from 1 mM to 10 mM and L-ascorbic acid in concentrations from 1 mM to 50 mM;
- treating a contaminated surface with said disinfecting composition, wherein microorganisms on said surface is killed by denaturing, solubilising inactivating and degrading their proteins and nucleic acids.

The microorganisms are killed with high efficiency by rubbing, wiping, spraying, soaking or immersion or in any other ways applying the composition solution to a contaminated surface contaminated with unwanted microorganisms. The solution should be applied to a surface at room temperature, and that surface to remain wet for the stated time period in order to kill the particular living microorganism being targeted from the surface being disinfected. In one embodiment the solution should be applied for about 1 minute. In one embodiment the solution should be applied for about 2 minutes. In one embodiment the solution should be applied for about 5 minutes. In one embodiment the solution should be applied for about 10 minutes. In one embodiment the solution should be applied for about 15 minutes. In one embodiment the solution should be applied for about 30 minutes. In one embodiment the solution should be applied for about 60 minutes. Depending on the surface and the specific task the applying method and applying time can be varied and adjusted.

In the following, exemplary disinfecting compositions according to the present invention will be described with reference to the figures and tables.

Figure 1 show that the present invention is able to targeting the following organisms: gram positive and gram negative bacteria, yeast and fungi, enveloped and non-enveloped viruses, mycobacteria and bacterial spores. "No efficacy" is indicated with a "no" and "full efficacy" is indicated with a "yes". The "Composition of the invention without Vitamin C" comprises 40 mM tartaric acid (CAS-No.: 87-69-4), 40 mM lactic acid (CAS-No.: 50-21-5), 3 mM CU-PCA (CAS-No.: 15454-74-7), 0.5% SDS (CAS-No.: 151-21-3), 7.9% EtOH (CAS-No.: 64-17-5), 4.4% PnB (CAS-No.: 5131-66-8), 0.99% glycerol (CAS-No.: 56-81-5), 0.099% Peppermint oil (CAS-No.: 8006-90-4) and 0.099% lavender oil (CAS-No.: 8000-28-0). The "Composition of the invention comprising Vitamin C" further comprises 40 mM L-ascorbic (CAS-No.: 50-81-7) acid and 10 mM NaCl (CAS-No.: 7647-14-5) (wipes) or 20 mM L-ascorbic (CAS-No.: 50-81-7) acid and 5 mM NaCl (CAS-No.: 7647-14-5) (fluid).

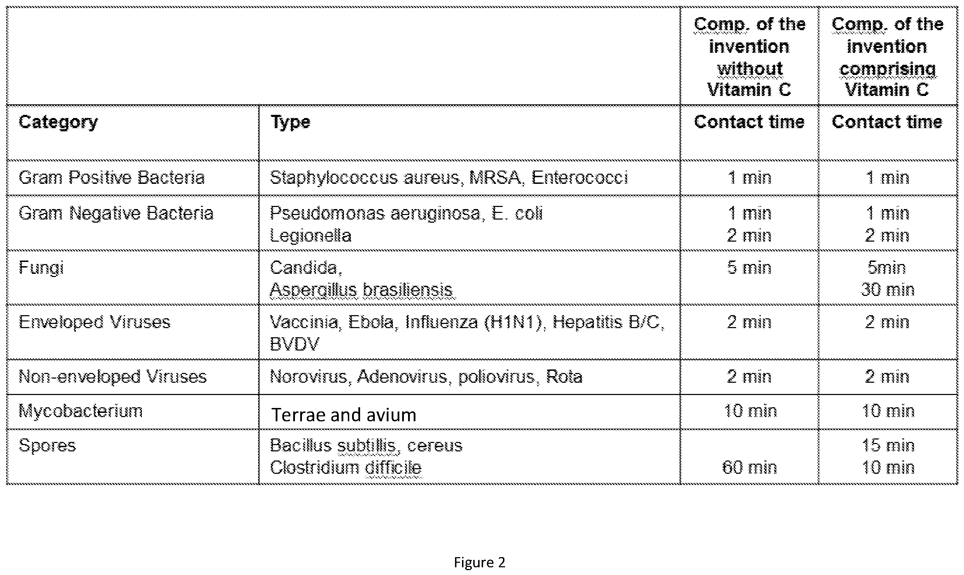

Efficacy tests of the present compositions were performed at around 20°C and the results are shown in figure 1 and the contact times are shown in figure 2.

Low level disinfectants like PHMB and DDAC are used on surfaces as a hygiene measure to prevent transmission of disease promoted by skin contact with contaminated surfaces.

Intermediate level disinfectants like ethanol, are used on surfaces as a preventing measure against organisms which cannot be killed by soap ans surface active disinfectant like PHMB and DDAC.

High-level disinfectants are used on surfaces during outbreak or visible contamination of organic matter i.e. blood and vomit. Some important target organisms are Aspergillus b., Bacillus S., Cereus and Clostridium spores, which can't be killed by alcohol, PHMB and DDAC.

EN 13727 is the *quantitative suspension test* for the evaluation of bactericidal activity in the medical area (phase 2, step 1), where the contact time is 1 minute.

EN 14561 is the *quantitative carrier test* for the evaluation of bactericidal activity of chemical disinfectants for instruments used in the medical area (phase 2, step 2) where the contact time is 1 minute.

EN 13624 is the *chemical disinfectants and antiseptics* - *quantitative suspension test* for the evaluation of fungicidal or yeasticidal activity in the medical area (phase 2, step 1), where the contact time is 5 minute for candida and 30 minutes for Aspergillus brasiliensis.

EN 14562 is the *quantitative carrier test* for the evaluation of fungicidal or yeasticidal activity for instruments used in the medical area (phase 2, step 2), where the contact time is 5 minutes for candida and 30 minutes for Aspergillus brasiliensis.

EN 14476 is the *chemical disinfectants and antiseptics* - *virucidal quantitative suspension test* for chemical disinfectants and antiseptic used in human medicine - Test method and requirements (phase 2, step 1), where the contact time is 2 minutes.

For the evaluation of the virucidal activity according to EN 14476 suspensions of 8 parts by volume of a 1.25% fold concentrated solution of the disinfectant solution (product test solution) with one volume of an interfering substance (0.3% in the test with dirty conditions and 0.03% for clean conditions) and one volume of virus suspension with a titer of at least 10 7 TC ID 50 / ml. To obtain a concentration of 97% 0,1ml virus, 0,2ml 5-fold concentrated loading and 9,7ml product were mixed. After the contact time the effect of the disinfectant will be stopped by diluting (or gel filtration). For the determination of residual virus content, a dilution series according to the progression of 1:10 / 1:100 / 1:1000 etc. will be performed. The titration is carried out in microtitre 96-well plates. In addition, the following controls are performed: virus control, cytotoxicity control, susceptibility control and a reference activation with 0.7% formaldehyde. The calculation is based on the method of Spear man and Kärber and is calculated as the difference between the titre of the virus control and the titre of product test solution including a 95% confidence interval.

EN 14348 is the *quantitative suspension test* for the evaluation of mycobactericidal activity of chemical disinfectants in the medical area including instrument disinfectants (phase 2, step 1), where the contact time is 10 minutes.

EN 14563 is the *quantitative carrier test* for the evaluation of mycobactericidal or tuberculocidal activity of chemical disinfectants used for instruments in the medical area (phase 2, step 2), where the contact time is 10 minutes.

EN 13704 is the *quantitative suspension test* for the evaluation of sporicidal activity of chemical disinfectants used in food, industrial, domestic and institutional areas (phase 2, step 1), where the contact time is 15 minutes for bacillus subtilis and cereus and 10 minutes for Clostridium difficile.

## Claims

1. Disinfecting composition comprising the following biocides:
a) Tartaric acid in concentrations from 1 mM to 70 mM;
b) Lactic acid in concentrations from 1 mM to 70 mM,
c)
1) lavender oil or lavender oil extract and
2) peppermint oil or peppermint oil extract in concentrations from 0.01 wt% to 1 wt%,
d) copper-PCA in concentrations from 1 mM to 10 mM.

2. Disinfecting composition according to claim 1, wherein the composition further comprises L-ascorbic acid.

3. Disinfecting composition according to claim 2, wherein L-ascorbic acid is included in concentrations from 1 mM to 50 mM.

4. Disinfecting composition according to any of the preceding claims, wherein the concentration of the lavender oil or lavender oil extract and peppermint oil or peppermint oil extract is from 0.05 wt% to 2 wt%, or from 0.1 wt% to 1 wt%, or from 0.1 wt% to 0.5 wt%.

5. Disinfecting composition according to any of the preceding claims, wherein the composition comprises from 0.01 wt% to 0.5 wt%, or from 0.05 wt% to 0.2 wt%, or from 0.09 wt% to 0.1 wt% lavender oil or lavender oil extract and from 0.01 wt% to 0.5 wt%, or from 0.05 wt% to 0.2 wt%, or from 0.09 wt% to 0.1 wt% peppermint oil or peppermint oil extract.

6. Disinfecting composition according to any of the preceding claims, wherein the concentration of the tartaric acid is from 10 mM to 50 mM, or from 20 mM to 50 mM, or from 30 mM to 50 mM, or from 35 mM to 45 mM, or 40 mM.

7. Disinfecting composition according to any of the preceding claims, wherein the concentration of the lactic acid is from 10 mM to 50 mM, or from 20 mM to 50 mM, or from 30 mM to 50 mM, or from 35 mM to 45 mM, or 40 mM.

8. Disinfecting composition according to any of the preceding claims, further comprising at least one buffer substances.

9. Disinfecting composition according to any of the preceding claims, further comprising alcohols.

10. Disinfecting composition according to claim 9, where the alcohol is Ethanol.

11. Disinfecting composition according to any of the preceding claims, having a pH value in the range of 2 to 4, or from 2.5 to 4, or from 2.5 to 3.

12. Use of the disinfecting composition according to any one of claims 1 to 11 for treating contaminated surfaces where the surfaces is selected from surfaces on fixed panel equipment and non-rinse/washable equipment such as control panels, medical instruments such as infusion pumps, stethoscopes, thermometers, blood pressure-apparatus, nanometres or flow meters, beds, nursing pillow, food preparation surfaces and equipment, walls or floors.

13. A disinfecting composition according to any one of claims 1 to 11 for use in a method for disinfection on skin such as hands and body.

## Patentansprüche

1. Desinfizierende Zusammensetzung, welche die folgenden Biozide umfasst:
a) Weinsäure in Konzentrationen von 1 mM bis 70 mM;
b) Milchsäure in Konzentrationen von 1 mM bis 70 mM,
c)
1) Lavendelöl oder Lavendelölextrakt und
2) Pfefferminzöl oder Pfefferminzölextrakt in Konzentrationen von 0,01 Gew.-% bis 1 Gew.-%,
d) Kupfer-PCA in Konzentrationen von 1 mM bis 10 mM.

2. Desinfizierende Zusammensetzung nach Anspruch 1, wobei die Zusammensetzung ferner L-Ascorbinsäure umfasst.

3. Desinfizierende Zusammensetzung nach Anspruch 2, wobei L-Ascorbinsäure in Konzentrationen von 1 mM bis 50 mM enthalten ist.

4. Desinfizierende Zusammensetzung nach einem der vorhergehenden Ansprüche, wobei die Konzentration des Lavendelöls oder Lavendelölextrakts und Pfefferminzöls oder Pfefferminzölextrakts von 0,05 Gew.-% bis 2 Gew.-% oder von 0,1 Gew.-% bis 1 Gew.-% oder von 0,1 Gew.-% bis 0,5 Gew.-% beträgt.

5. Desinfizierende Zusammensetzung nach einem der vorhergehenden Ansprüche, wobei die Zusammensetzung von 0,01 Gew.-% bis 0,5 Gew.-% oder von 0,05 Gew.-% bis 0,2 Gew.-% oder von 0,09 Gew.-% bis 0,1 Gew.-% Lavendelöl oder Lavendelölextrakt und von 0,01 Gew.-% bis 0,5 Gew.-% oder von 0,05 Gew.-% bis 0,2 Gew.-% oder von 0,09 Gew.-% bis 0,1 Gew.-% Pfefferminzöl oder Pfefferminzölextrakt umfasst.

6. Desinfizierende Zusammensetzung nach einem der vorhergehenden Ansprüche, wobei die Konzentration der Weinsäure von 10 mM bis 50 mM oder von 20 mM bis 50 mM oder von 30 mM bis 50 mM oder von 35 mM bis 45 mM oder 40 mM beträgt.

7. Desinfizierende Zusammensetzung nach einem der vorhergehenden Ansprüche, wobei die Konzentration der Milchsäure von 10 mM bis 50 mM oder von 20 mM bis 50 mM oder von 30 mM bis 50 mM oder von 35 mM bis 45 mM oder 40 mM beträgt.

8. Desinfizierende Zusammensetzung nach einem der vorhergehenden Ansprüche, die ferner mindestens eine Puffersubstanz umfasst.

9. Desinfizierende Zusammensetzung nach einem der vorhergehenden Ansprüche, die ferner Alkohole umfasst.

10. Desinfizierende Zusammensetzung nach Anspruch 9, wobei der Alkohol Ethanol ist.

11. Desinfizierende Zusammensetzung nach einem der vorhergehenden Ansprüche, die einen pH-Wert in dem Bereich von 2 bis 4 oder von 2,5 bis 4 oder von 2,5 bis 3 aufweist.

12. Verwendung der desinfizierenden Zusammensetzung nach einem der Ansprüche 1 bis 11 zum Behandeln von kontaminierten Oberflächen, wobei die Oberflächen aus Oberflächen auf fest eingebauter Ausrüstung und nicht spül-/abwaschbarer Ausrüstung wie etwa Bedienfeldern, medizinischen Instrumenten wie etwa Infusionspumpen, Stethoskopen, Thermometern, Blutdruckmessgeräten, Nanometern oder Durchflussmessern, Betten, Pflegekissen, Oberflächen und Ausrüstung für die Lebensmittelzubereitung, Wänden oder Böden ausgewählt sind.

13. Desinfizierende Zusammensetzung nach einem der Ansprüche 1 bis 11 zur Verwendung in einem Verfahren zur Desinfektion von Haut wie etwa Händen und Körper.

## Revendications

1. Composition désinfectante comprenant les biocides suivants :
a) acide tartrique à des concentrations de 1 mM à 70 mM ;
b) acide lactique à des concentrations de 1 mM à 70 mM,
c)
1) huile de lavande ou extrait d'huile de lavande et
2) huile de menthe poivrée ou extrait d'huile de menthe poivrée à des concentrations de 0,01 % en poids à 1 % en poids,
d) cuivre-PCA à des concentrations de 1 mM à 10 mM.

2. Composition désinfectante selon la revendication 1, dans laquelle la composition comprend en outre de l'acide L-ascorbique.

3. Composition désinfectante selon la revendication 2, dans laquelle l'acide L-ascorbique est inclus à des concentrations de 1 mM à 50 mM.

4. Composition désinfectante selon l'une quelconque des revendications précédentes, dans laquelle la concentration de l'huile de lavande ou de l'extrait d'huile de lavande et de l'huile de menthe poivrée ou de l'extrait d'huile de menthe poivrée est de 0,05 % en poids à 2 % en poids, ou de 0,1 % en poids à 1 % en poids, ou de 0,1 % en poids à 0,5 % en poids.

5. Composition désinfectante selon l'une quelconque des revendications précédentes, dans laquelle la composition comprend de 0,01 % en poids à 0,5 % en poids, ou de 0,05 % en poids à 0,2 % en poids, ou de 0,09 % en poids à 0,1 % en poids d'huile de lavande ou d'extrait d'huile de lavande et de 0,01 % en poids à 0,5 % en poids, ou de 0,05 % en poids à 0,2 % en poids, ou de 0,09 % en poids à 0,1 % en poids d'huile de menthe poivrée ou d'extrait d'huile de menthe poivrée.

6. Composition désinfectante selon l'une quelconque des revendications précédentes, dans laquelle la concentration de l'acide tartrique est de 10 mM à 50 mM, ou de 20 mM à 50 mM, ou de 30 mM à 50 mM, ou de 35 mM à 45 mM, ou de 40 mM.

7. Composition désinfectante selon l'une quelconque des revendications précédentes, dans laquelle la concentration de l'acide lactique est de 10 mM à 50 mM, ou de 20 mM à 50 mM, ou de 30 mM à 50 mM, ou de 35 mM à 45 mM, ou de 40 mM.

8. Composition désinfectante selon l'une quelconque des revendications précédentes, comprenant en outre au moins une substance tampon.

9. Composition désinfectante selon l'une quelconque des revendications précédentes, comprenant en outre des alcools.

10. Composition désinfectante selon la revendication 9, où l'alcool est l'éthanol.

11. Composition désinfectante selon l'une quelconque des revendications précédentes, ayant une valeur de pH dans la plage de 2 à 4, ou de 2,5 à 4, ou de 2,5 à 3.

12. Utilisation de la composition désinfectante selon l'une quelconque des revendications 1 à 11 pour traiter des surfaces contaminées où les surfaces sont choisies parmi les surfaces sur un équipement à panneaux fixes et un équipement non rinçable/lavable tel que des panneaux de commande, des instruments médicaux tels que des pompes à perfusion, des stéthoscopes, des thermomètres, des appareils de mesure de la tension artérielle, des nanomètres ou des débitmètres, des lits, des coussins d'allaitement, des surfaces et des équipements de préparation des aliments, des murs ou des sols.

13. Composition désinfectante selon l'une quelconque des revendications 1 à 11 destinée à être utilisée dans un procédé de désinfection de la peau telle que celle des mains et du corps.
